# EUROPEAN PATENT SPECIFICATION

(11) **EP 1 861 033 B1**
(45) Date of publication and mention of the grant of the patent: **13.06.2012**
(21) Application number: 05714715.9
(22) Date of filing: 24.03.2005
(51) Int. Cl.: A61B 17/70, A61B 17/86, A61B 17/88, A61B 17/68

(54) **DEVICE FOR THE CEMENT AUGMENTATION OF BONE IMPLANTS**
VORRICHTUNG ZUR ZEMENTVERSTÄRKUNG VON KNOCHENIMPLANTATEN
APPAREIL POUR L'EXPANSION DU CIMENT D'IMPLANTS OSSEUX

(43) Date of publication of application: 05.12.2007
(73) Proprietor: Synthes GmbH, 4436 Oberdorf (CH)
(72) Inventor: FRIGG, Robert, CH-2544 Bettlach (CH)
(74) Representative: Lusuardi, Werther
(86) International application number: PCT/CH2005/000173
(87) International publication number: WO 2006/099751

(56) References cited:
- DE-A1- 19 605 735
- DE-U1- 29 501 042
- US-A- 5 755 720
- PATENT ABSTRACTS OF JAPAN vol. 2003, no. 10, 8 October 2003 (2003-10-08) -& JP 2003 159258 A (OKUTSU ICHIRO), 3 June 2003 (2003-06-03)

## Description

The invention relates to a device for the cement augmentation of bone implants according to the preamble of claim 1.

Bone cement augmentation in bone surgery is known for several years. This augmentation is applied in osteoporotic bones where bone anchoring means as screws, pins, nails and so on may not anchored rigidly.

According to the known technology the bone cement is inserted into the bone and is suitable as a anchoring bed for subsequently inserted implants. Under bone cement every bone filling material is understood that is hardenable by means of polymer, hydraulic or according to other reaction mechanisms. These are inserted before the cement hardens such that the cement may add on the implants. The drawback of this known technology is the complicated application of the bone cement and the determination of the correct position of the implant within the cement composite.

In order to solve these problems particular implants have been developed that allow an application of the cement when the implant is lying. Thereto, implants and particularly screws haven been used which comprise a cannulation wherethrough the cement may be filled into the bone. This technology requires a high viscous cement which may be injected through the relatively small cannulation. Furthermore, the amount and the position of the cement in the bone may not be controlled sufficiently. Bone cements having a lower viscosity which have significant biological advantages with regard to bone cements that are not resorbable and that harden at a high temperature are not suitable in combination with these adapted implants.

From DE-A-3 508 759 TRONZO for example such a hip screw is known which is provided with a central through bore, said through bore opening in an open screw tip and having sideward openings between the thread walls. By means of this hip screw an anchorage of the oversized and pointed thread in the bone shall be achieved on the one side and on the other side a strengthening of the weakened bone material shall be achieved by means of injecting bone cements through the central bore and outflow through the sideward openings. The main disadvantage of this technology is to be seen In the fact, that no common bone implants may be used but a especially manufactured, costly and complicated implant (hip screw) are necessary.

From DE 196 05 735 A1 KUMM a device is known comprising a hollow cylinder and a bone implant in the form of a member of an endoprosthesis with a cross-section corresponding to the opening of the hollow cylinder. The bone implant is insertable in the hollow cylinder which is either open or close on its front end. Furthermore, the cylinder is provided with radial perforations in the form of a grid mesh and prefilled with bone cement.

From US 5,755,720 MIKHAIL et al. a device is known comprising a hollow sleeve prefilled with a bone cement and a member of an endoprosthesis being insertable into the sleeve, whereby the sleeve comprises an opening corresponding to the cross-section of the bone implant; said sleeve further being provided with perforations in the form of a grid mesh. Furthermore, this device comprises an injection apparatus for pre-filling the hollow cylinder with bone cement and the hollow cylinder comprises an adapter for connection with the injection apparatus. Additionally, this document discloses different thicknesses of the sleeve in the range of 0.5 to 3.0 mm and different diameters of the perforations in the range of 1.0 to 3.0 mm.

JP 2003-159258 OKUTSU discloses a hollow body being provided with a plurality of perforations and comprising a central cavity for injection of bone cement. OKUTSU does not comprise a bone implant and a hollow sleeve, but solely comprises a bone implant provided with apertures.

On this point, the invention intends to provide remedial measures. The invention is based on the objective of providing a device by means of which the cement augmentation of existing and/or novel bone screws may be simplified.

The invention solves the posed problem with a device for cement augmentation that displays the features of claim 1.

The device according to the invention separates the particular augmentation from the bone screw that is to be augmented, wherethrough the intra-operative handling is simplified and the security of this treatment technology is increased.

In case of the invention a perforated hollow cylinder prefilled with bone cement is inserted in the bone. Depending on the situation the seat for the hollow cylinder in the bone has to be prepared. This may be effected through boring and/or reaming. Once the hollow cylinder has been seated the bone screw corresponding to the hollow cylinder is inserted in the hollow cylinder therewith displacing the cement through the perforations in the hollow cylinder in surrounding bone. The amount of the flown out cement equals the volume of the anchoring means. Depending of the position and number of perforations in the hollow cylinder the augmentation may be effected in a desired region. Thanks to the separation of the augmentation device for the bone screw a cement augmentation may be effected by using usual commercial bone screws.

The application of the cement is not effected complicatedly in the bone as in case of the known augmentation but is effected in a controlled manner on the operating table into the hollow cylinder. The hollow cylinder may be inserted in the prepared seat in the bone once the filling has been effected.

Basically the hollow cylinder may be applied in every situation where the bone material does not give enough rigidity for common bone screws, for example in the regions near a bone joint, the vertebra, jawbone, pelvis and so on.

The advantages achieved by the invention are manifold:
- application of the cement exterior of the body
   i) simple handling of the cement
   ii) filling procedure being controllable and doseable
   iii) no inclusion of blood, liquid and tissue in the cement
- through orientation, position, number and dimension of the perforation the cement is lead to the anatomically desired location;
- the amount of cement emerging of the hollow cylinder may be controlled via the volume of the bone screw ; and
- the risk of uncontrolled loss of a large amount of cement in the fracture region because of non-controllable decrease of resistance (spontaneous bone fracture in the treatment area) is prevented since the emerging amount of cement is controlled via the insertion of the bone screw and not via a applied pressure against the resistance of the bone.

In a preferred embodiment of the invention the hollow cylinder is at least partially filled with not yet hardened bone cement.

In a further embodiment the hollow cylinder and the bone screw are mutually adapted such that upon insertion of the bone screw, in the hollow cylinder the not yet hardened bone cement flows out through the at least one perforation in the shell of the hollow cylinder.

In a further embodiment the hollow cylinder is closed at its front end. The advantage achieved therewith is that the cement flows out only sidewardly where it is most suitable since a hardened cluster of cement in front of the hollow cylinder might be disadvantageous since upon further screwing in of the bone screw the cluster of cement might penetrate into the corticalis being in front of it. In case of TRONZO for example the bone screw may not be screwed in axial direction towards the front after hardening of the cement since the cement flown out through the open tip and having hardened does not allow this. An additional advantage of the hollow cylinder being closed at its front end is achieved in case of its application near a bone joint.

In a further embodiment the rear end of the hollow cylinder is configured as a coupling for the connection with syringe for bone cement. Especially, the rear end of the hollow cylinder may be a plug-in connection for an adapter for coupling with a syringe for bone cement. This impermeable connection prevents on the one side a loss of pressure and on the other side an undesired flow out of bone cement at the rear end of the hollow cylinder.

In a further embodiment the rear end of the hollow cylinder is provided with an aperture having the cross-section of the bone screw, e.g. in the form of a screw thread.

In a further embodiment the shell is provided with the at least one perforation on a partial length of maximum L/3 measured from the front end. Such bone cement is flowing out only in the frontal region of the hollow cylinder where it offers the outmost advantage. The flowing out of bone cement in the rear portion is clinically rather disadvantageous.

In a further embodiment the rear end of the hollow cylinder is configured as a cavity with polygonal cross-section or TORX-cavity allowing the acceptance of a respective polygonal- or TORX screw driver.

The diameter of the at least one perforation is typically in the range between 0,9 mm and 3,3 mm. The number of perforations is typically at least 20, preferably at least 40. The maximum number of perforations is typically maximum 100, preferably maximum 60. Preferably, the ratio D/F between the outer diameter of the hollow cylinder in mm and the total outflow area F of the perforations in mm² is in the range between 0,19 and 0,36 mm⁻¹.

The wall thickness (outer diameter D minus inner diameter d) of the hollow cylinder is preferably in the range between 0,1 and 2,0 mm.

As materials the common implant materials as steel or titan are suitable, but synthetics as PEEK or resorbable or non-resorbable polymers may be used as well. The hollow cylinder may manufactured of a mesh, a braiding or a fully or partially perforated tube. By means of the position, diameter and number of perforations the outflow of cement may be controlled with respect to location and amount and adjusted to the cement being used.
The face of the front end may be open or closed depending on the requirements. The closed embodiment prevents a facial outflow of the cement, which is important for some applications (application near a bone joint).

Additional advantageous embodiments of the invention are characterized in the subclaims.

The invention and additional configurations of the invention are explained in even more detail with reference to the partially schematic illustration of several embodiments.

Shown are:
Fig. 1 a perspective view of a hollow cylinder for the device according to the invention;
Fig. 2 a perspective view of the hollow cylinder according to fig. 1 and a longitudinal section through a cement syringe for pressing the bone cement in the hollow cylinder;
Fig. 3 a perspective view of the hollow cylinder filled with bone cement according to fig. 1 whereby a bone screw is inserted, said bone screw pressing a portion of the bone cement in the surrounding bone;
Fig. 4 a longitudinal section through empty hollow cylinder according to fig. 1;
Fig. 5 a longitudinal section through the hollow cylinder according to fig. 1 filled with bone cement and a bone cement syringe;
Fig. 6 a longitudinal section through the hollow cylinder according to fig. 1 filled with bone cement and a bone screw inserted in the hollow cylinder, said bone screw pressing a portion of the bone cement in the surrounding bone.

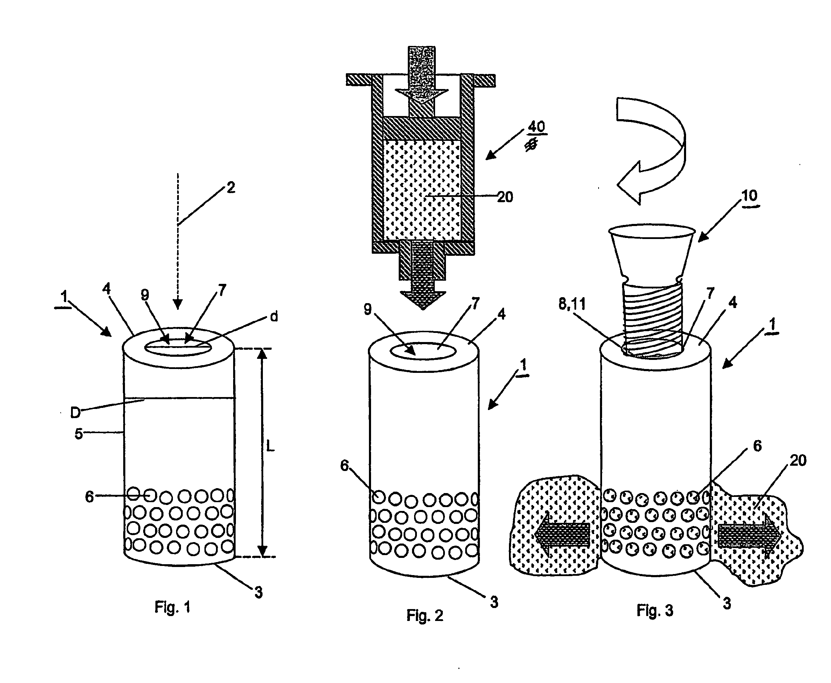

The embodiment shown in fig. 1 essentially comprises a hollow cylinder 1 with a front end 3, a rear end 4 and a longitudinal axis 2. The hollow cylinder 1 has a total length L extending parallel to the longitudinal axis 2 and an outer diameter D measured orthogonally to the longitudinal axis 2. The cavity 9 of the hollow cylinder 9 has an inner diameter d measured orthogonally to the longitudinal axis 2. The cavity 9 of the hollow cylinder 1 is enclosed by a shell 5 along the total length L, whereby the shell 5 is provided partially with perforations 6. The total of all perforations 6 sums up to a total outlet area F which is smaller than the surface of the shell 5.

The front end 3 of the hollow cylinder 1 may be open or closed, the closed embodiment being advantageous since the formation of cement clusters in front of the hollow cylinder 1 may be prevented. At its rear end 4 the hollow cylinder 1 is provided with a bore hole 7 opening into the cavity 9 such that it suits as inlet for a bone cement. The bore hole 7 is provided with an interior thread 8 (fig. 3).

The embodiment shown in fig. 2 comprises a hollow cylinder 1 having a shell 5 which is provided with perforations 6, further having a front end 3 and a rear end 4. A bore hole 7 penetrates the hollow cylinder 1 from the front end 3, said bore hole 7 being apt to insert the bone cement 20 from a cement syringe 40 (longitudinal section) into the cavity 9 of the hollow cylinder 1.

Fig. 3 comprises a hollow cylinder 1 filled with bone cement 20 and having a closed front end 3. At the rear end 4 the bore hole 7 is provided with an interior thread 8 in the hollow cylinder 1. The bone implant 10 is here configured as a bone anchoring element 15 in the form of a bone screw. The interior thread 8 matches with the exterior thread of the bone anchoring element 15 such that the bone anchoring element 15 is screwable into the hollow cylinder 1. Since the shell 5 is provided with perforations 6 a portion of the bone cement 20 is pressed radially through the perforations 6 out of the hollow cylinder 1 by means of the bone anchoring element 15.

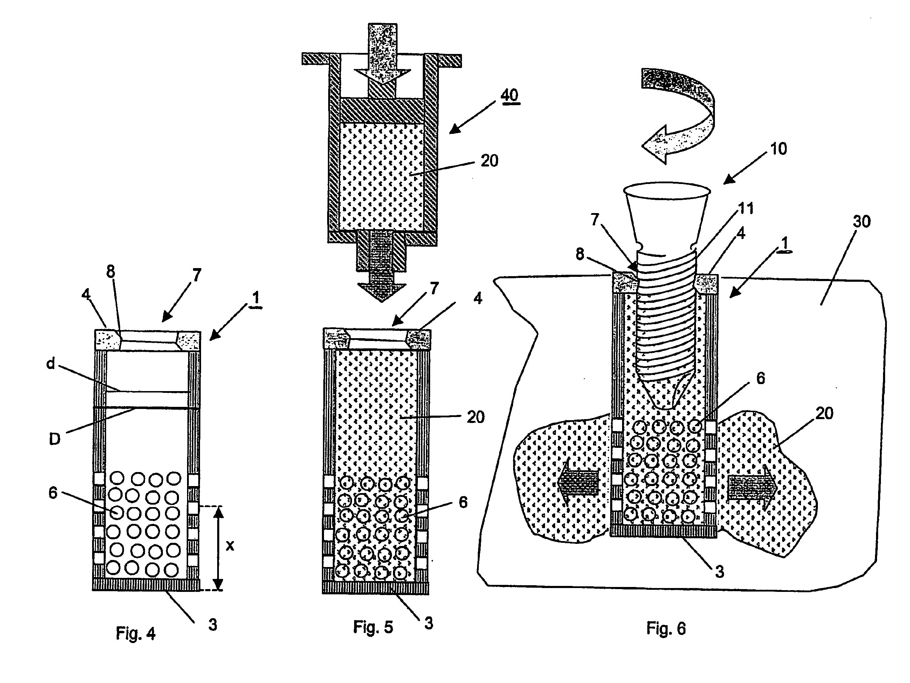

The embodiment shown in fig. 4 depicts a longitudinal section through the empty hollow cylinder 1 according to fig. 1, whereby the front end 3 of the hollow cylinder 1 is closed and the bore hole 7 is provided with an interior thread 8 at the rear end 4. The hollow cylinder 1 has a outer diameter D and an inner diameter d. The shell 5 of the hollow cylinder 1 is provided with perforations 6 on a partial length X < L measured from the front end 3.

Fig. 5 shows a longitudinal section through a hollow cylinder 1 filled with bone cement 20 and having a closed front end 3 as well as a bore hole 7 at the rear end 4. Furthermore, fig. 5 depicts a longitudinal section through a bone cement syringe 40 filled with bone cement 20.

Fig. 6 depicts an embodiment in a longitudinal section through the hollow cylinder 1 filled with bone cement 20, said hollow cylinder 1 having a closed front end 3 and a rear end 4 being provided with a bore hole 7. A bone anchoring element 15 is inserted in the bore hole 7 at the rear end 4, said bone anchoring element 15 being provided with a exterior thread 11 which matches with the interior thread 8 in the bore hole 7. By means of inserting the bone anchoring means 15 the bone cement 20 is pressed out of the hollow cylinder 1 through the perforations 6 in the enclosing bone 30.

In the following the surgical technique for the device for cement augmentation of bone implants according to the invention is shortly described:
a) the seat of the hollow cylinder is being prepared in the bone. This is effected e.g. by means of reaming the bone;
b) the hollow cylinder being chosen with respect to its length and diameter is being filled with bone cement - in a common manner externally of the patient's body;
c) in order to prevent an outflow of the bone cement through the perforations of the cylinder shell, the hollow cylinder may for example inserted in a socket having a corresponding bore;
d) the pre-filled hollow cylinder is inserted in the bone;
e) the bone screw is inserted into the hollow cylinder through the rear end of the hollow cylinder. During this procedure the bone cement is pressed through the perforations in the bone structure enclosing the hollow cylinder;
f) after the bone cement has hardened the bone cement generates a unit comprising the bone, the hollow cylinder and the bone screw.

## Claims

1. Device for cement augmentation of bone implants, with
a hollow cylinder (1) being suitable for the acceptance of a not yet hardened bone cement (20), said hollow cylinder (1) having a longitudinal axis (2), an interior thread (8), a front end (3) being suitable for insertion into a bone (30), a rear end (4) and a shell (5) with at least one perforation (6); and
a screw (10) with an exterior thread (11) being insertable into the hollow cylinder (1) from the rear end (4) thereof, whereby said exterior thread (11) corresponds to said interior thread (8) of the hollow cylinder (1),
**characterized in that**
said screw is a bone screw and said hollow cylinder (1) has a total length L, wherein the shell (5) is provided with said at least one perforation (6) on a partial length of maximum L/2 measured from the front end (3).

2. Device according to claim 1, wherein the hollow cylinder (1) is filled at least partially with not yet hardened bone cement (20).

3. Device according to claim 1 or 2, wherein the hollow cylinder (1) and the bone screw (10) are mutually matched such that upon insertion of the bone screw (10) into the hollow cylinder (1) the not yet hardened bone cement (20) penetrates the at least one perforation (6) in the shell (5) of the hollow cylinder (1).

4. Device according to one of the claims 1 to 3, wherein the hollow cylinder (1) is closed at its front end (3).

5. Device according to one of the claims 1 to 4, wherein the rear end (4) of the hollow cylinder (1) is configured as a coupling for the connection with a bone cement syringe (40).

6. Device according to one of the claims 1 to 5, wherein the rear end (4) of the hollow cylinder (1) is configured as a plug-in connection for an adapter for the coupling with a bone cement syringe (40).

7. Device according to one of the claims 1 to 6, wherein the rear end (4) of the hollow cylinder (1) is provided with an opening that corresponds to the cross-section of the bone srew (10).

8. Device according to one of the claims 1 to 7, wherein the shell is provided with the at least one perforation (6) on a partial length of maximum L/3 measured from the front end.

9. Device according to one of the claims 1 to 8, wherein the rear end (4) of the hollow cylinder (1) is configured as polygonal cavity or TORX-cavity for the acceptance of a respective polygonal or TORX-screw driver.

10. Device according to one of the claims 1 to 9, wherein the diameter of the perforations (6) is between 0,9 - 3,3 mm, preferably between 1,5 - 2,5 mm.

11. Device according to one of the claims 1 to 10, wherein the number of perforations (6) amounts to at least 20, preferably to at least 40.

12. Device according to one of the claims 1 to 11, wherein the number of perforations (60) amounts to maximum 100, preferably to maximum 60.

13. Device according to one of the claims 1 to 12, wherein the ratio D/F between the outer diameter D of the hollow cylinder (1) measured in mm and the total outlet area F of the perforations (6) measured in mm² is in the range of 0,19 and 0,36 mm⁻¹.

14. Device according to one of the claims 1 to 13, wherein the wall thickness (outer diameter minus inner diameter) of the hollow cylinder (1) is in the range of 0,1 and 2,0 mm.

15. Device according to one of the claims 1 to 14, wherein the perforations (6) have the form of a grid mesh.

## Patentansprüche

1. Vorrichtung zur Verstärkung von Knochenimplantaten mittels Zement, mit
einem Hohlzylinder (1), welcher zur Aufnahme eines noch nicht ausgehärteten Knochenzements (20) geeignet ist, wobei der Hohlzylinder (1) eine Längsachse (2), ein Innengewinde (8), ein zur Einführung in einen Knochen geeignetes vorderes Ende (3), ein hinteres Ende (4) und eine Schale (5) mit mindestens einer Perforation (6) hat; und
einer Schraube (10) mit einem Aussengewinde (11), welche vom hinteren Ende (4) des Hohlzylinders (1) in diesen einführbar ist, wobei das Aussengewinde (11) zum Innengewinde (8) des Hohlzylinders (1) korrespondiert,
**dadurch gekennzeichnet, dass**
die Schraube eine Knochenschraube ist und der Hohlzylinder (1) eine Gesamtlänge L hat, wobei
die Schale (5) auf einer vom vorderen Ende (3) gemessenen Teillänge von höchstens U2 mit der mindestens einen Perforation (6) versehen ist.

2. Vorrichtung nach Anspruch 1, **dadurch gekennzeichnet, dass** der Hohlzylinder (1) mindestens teilweise mit noch nicht ausgehärtetem Knochenzement gefüllt ist.

3. Vorrichtung nach Anspruch 1 oder 2, **dadurch gekennzeichnet, dass** der Hohlzylinder (1) und die Knochenschraube (10) zueinander passen, so dass beim Einsetzen der Knochenschraube (10) in den Hohlzylinder (1) der noch nicht ausgehärtete Knochenzement (20) durch die mindestens eine Perforation (6) in der Schale (5) des Hohlzylinders (1) penetriert.

4. Vorrichtung nach einem der Ansprüche 1 bis 3, **dadurch gekennzeichnet, dass** der Hohlzylinder (1) an seinem vorderen Ende (3) geschlossen ist.

5. Vorrichtung nach einem der Ansprüche 1 bis 4, **dadurch gekennzeichnet, dass** das hintere Ende (4) des Hohlzylinders (1) als Kupplung zur Verbindung mit einer Knochenzementspritze (40) ausgebildet ist.

6. Vorrichtung nach einem der Ansprüche 1 bis 5, **dadurch gekennzeichnet, dass** das hintere Ende (4) des Hohlzylinders (1) als Steckverbindung für einen Adapter zur Kupplung an eine Knochenzementspritze (40) ausgebildet ist.

7. Vorrichtung nach einem der Ansprüche 1 bis 6, **dadurch gekennzeichnet, dass** das hintere Ende (4) des Hohlzylinders (1) mit einer Öffnung versehen ist, welche mit dem Querschnitt der Knochenschraube (10) korrespondiert.

8. Vorrichtung nach einem der Ansprüche 1 bis 7, **dadurch gekennzeichnet, dass** die Schale auf einer vom vorderen Ende gemessenen Teillänge von höchstens L/3 mit der mindestens einen Perforation (6) versehen ist.

9. Vorrichtung nach einem der Ansprüche 1 bis 8, **dadurch gekennzeichnet, dass** das hintere Ende (4) des Hohlzylinders (1) als polygonale Kavität oder TORX-Kavität zur Aufnahme eines entsprechenden polygonalen oder TORX-Schraubendrehers ausgebildet ist.

10. Vorrichtung nach einem der Ansprüche 1 bis 9, **dadurch gekennzeichnet**, **dadurch gekennzeichnet, dass** der Durchmesser der Perforationen (6) zwischen 0,9 mm - 3,3 mm, vorzugsweise zwischen 1,5 mm - 2,5 mm beträgt.

11. Vorrichtung nach einem der Ansprüche 1 bis 10, **dadurch gekennzeichnet**, **dadurch gekennzeichnet, dass** die Anzahl Perforationen (6) mindestens 20, vorzugsweise mindestens 40 beträgt.

12. Vorrichtung nach einem der Ansprüche 1 bis 11, **dadurch gekennzeichnet, dass** die Anzahl Perforationen (6) maximal 100, vorzugsweise maximal 60 beträgt.

13. Vorrichtung nach einem der Ansprüche 1 bis 12, **dadurch gekennzeichnet, dass** das Verhältnis D / F zwischen dem Aussendurchmesser D des Hohlzylinders (1) in mm gemessen und der gesamten Auslassfläche F der Perforationen (6) in mm² gemessen im Bereich zwischen 0,19 und 0,36 mm⁻¹ liegt.

14. Vorrichtung nach einem der Ansprüche 1 bis 13, **dadurch gekennzeichnet, dass** die Wanddicke (Aussendurchmesser minus Innendurchmesser) des Hohlzylinders (1) im Bereich zwischen 0,1 mm bis 2,0 mm liegt.

15. Vorrichtung nach einem der Ansprüche 1 bis 14, **dadurch gekennzeichnet, dass** die Perforationen (6) die Form eines Maschengitters aufweisen.

## Revendications

1. Dispositif d'augmentation par ciment d'implants osseux, comprenant :
un cylindre creux (1) conçu pour recevoir un ciment osseux n'ayant pas encore durci (20), ledit cylindre creux (1) ayant un axe longitudinal (2), un filetage intérieur (8), une extrémité avant (3) conçue pour être insérée dans un os (30), une extrémité arrière (4) et une enveloppe (5) pourvue d'au moins une perforation (6) ; et
une vis (10) à filetage extérieur (11) pouvant être insérée dans le cylindre creux (1) par l'extrémité arrière (4) de celui-ci, ledit filetage extérieur (11) correspondant audit filetage intérieur (8) du cylindre creux (1),
**caractérisé en ce que**
ladite vis est une vis à os et ledit cylindre creux (1) a une longueur totale L,
et **en ce que** l'enveloppe (5) est pourvue de ladite au moins une perforation (6) sur une longueur partielle d'au maximum L/2, mesurée depuis l'extrémité avant (3).

2. Dispositif selon la revendication 1, **caractérisé en ce que** le cylindre creux (1) est rempli au moins en partie de ciment osseux n'ayant pas encore durci (20).

3. Dispositif selon la revendication 1 ou 2, **caractérisé en ce que** le cylindre creux (1) et la vis à os (10) sont mutuellement appariés de sorte que, lors de l'insertion de la vis à os (10) dans le cylindre creux (1), le ciment osseux n'ayant pas encore durci (20) pénètre dans la au moins une perforation (6) formée dans l'enveloppe (5) du cylindre creux (1).

4. Dispositif selon l'une des revendications 1 à 3, **caractérisé en ce que** le cylindre creux (1) est fermé au niveau de son extrémité avant (3).

5. Dispositif selon l'une des revendications 1 à 4, **caractérisé en ce que** l'extrémité arrière (4) du cylindre creux (1) est conçue sous la forme d'un raccord pour l'accouplement d'une seringue à ciment osseux (40).

6. Dispositif selon l'une des revendications 1 à 5, **caractérisé en ce que** l'extrémité arrière (4) du cylindre creux (1) est conçue sous la forme d'un raccord enfichable pour adaptateur pour l'accouplement d'une seringue à ciment osseux (40).

7. Dispositif selon l'une des revendications 1 à 6, **caractérisé en ce que** l'extrémité arrière (4) du cylindre creux (1) est pourvue d'une ouverture qui correspond à la section droite de la vis à os (10).

8. Dispositif selon l'une des revendications 1 à 7, **caractérisé en ce que** l'enveloppe est pourvue de la au moins une perforation (6) sur une longueur partielle d'au maximum U3, mesurée depuis l'extrémité avant.

9. Dispositif selon l'une des revendications 1 à 8, **caractérisé en ce que** l'extrémité arrière (4) du cylindre creux (1) est conçue sous la forme d'une cavité polygonale ou d'une cavité TORX pour recevoir un tournevis polygonal ou TORX respectif.

10. Dispositif selon l'une des revendications 1 à 9, **caractérisé en ce que** le diamètre des perforations (6) est compris entre 0,9 et 3,3 mm, de préférence entre 1,5 et 2,5 mm.

11. Dispositif selon l'une des revendications 1 à 10, **caractérisé en ce que** le nombre de perforations (6) s'élève à au moins 20, de préférence à au moins 40.

12. Dispositif selon l'une des revendications 1 à 11, **caractérisé en ce que** le nombre de perforations (6) s'élève au maximum à 100, de préférence au maximum à 60.

13. Dispositif selon l'une des revendications 1 à 12, **caractérisé en ce que** le rapport D / F entre le diamètre extérieur D du cylindre creux (1), mesuré en mm, et la surface de sortie totale F des perforations (6), mesurée en mm², se situe dans la gamme de 0,19 à 0,36 mm⁻¹.

14. Dispositif selon l'une des revendications 1 à 13, **caractérisé en ce que** l'épaisseur de paroi (diamètre extérieur moins diamètre intérieur) du cylindre creux (1) se situe dans la gamme de 0,1 à 2,0 mm.

15. Dispositif selon l'une des revendications 1 à 14, **caractérisé en ce que** les perforations (6) ont la forme d'un treillis.
